# EUROPEAN PATENT APPLICATION

(11) **EP 2 085 417 A1**
(43) Date of publication of application: **05.08.2009**
(21) Application number: 07831594.2
(22) Date of filing: 06.11.2007
(51) Int. Cl.: C08G 63/183, C07C 69/82

(54) **METHOD FOR PRODUCING TEREPHTHALIC ACID-ALKYLENE GLYCOL MIXTURE**

(30) Priority: 07.11.2006 JP 2006301376
(71) Applicant: Teijin Fibers Limited, Osaka-shi, Osaka 541-0054 (JP)
(72) Inventor: TOIDA, Kozo, Matsuyama-shi Ehime 791-8041 (JP); NAKASHIMA, Minoru, Matsuyama-shi Ehime 791-8041 (JP)
(74) Representative: Hallybone, Huw George
(86) International application number: PCT/JP2007/071864
(87) International publication number: WO 2008/056801

(57) **Abstract**

A liquid mixture of terephthalic acid with an alkylene glycol suitable as a starting material mixture for a polyester polycondensation reaction is produced by preparing a liquid mixture of terephthalic acid particles prepared by the hydrolysis of a dialkyl terephthalate with at least one type of alkylene glycol and having a temperature of 100 to 130°C, and then stirring the liquid mixture for 15 minutes to 3 hours while the temperature of the liquid mixture is maintained in the above-mentioned range, to control the slurry properties of the liquid mixture.

## Description

### TECHNICAL FIELD

The present invention relates to a process for producing a liquid mixture of terephthalic acid with an alkylene glycol as a raw material for producing a polyester resin, typically a polyethylene terephthalate resin, which is widely used for fibers, films, industrial parts, shaped articles, etc. More particularly, the present invention relates to a process for producing a liquid mixture of terephthalic acid, which is substantially produced by hydrolysis of dimethyl terephthalate, with an alkylene glycol, which has good slurry properties and is suitable as a raw material for the production of a polyester resin, typically a polyethylene terephthalate resin.

### BACKGROUND ART

A polyester such as polyethylene terephthalate (PET) is produced through a slurry preparation step in which a dicarboxylic acid (typically terephthalic acid) is mixed with an appropriate alkylene glycol (typically ethylene glycol), an esterification step in which the dicarboxylic acid is reacted with the alkylene glycol to prepare a bis(ω-hydroxyalkyl) ester and a slight amount of a linear oligomer, a polycondensation step in which the esterification step product is polycondensed to increase the molecular weight, and a productization step in which the resultant polymer is crystallized and granulated. In the slurry preparation step of this process, a dicarboxylic acid such as terephthalic acid is dispersed and suspended in an alkylene glycol such as ethylene glycol to form a slurry, and then in the esterification step, the dicarboxylic acid in the slurry is esterified through a reaction with the alkylene glycol. Furthermore, the product obtained in the above-described esterification step is polycondensed to produce a polyester in the polycondensation step, and the obtained polyester is subjected to crystallization and granulation treatments to obtain a finished product in the productization step.

Enhancement of efficiency in the slurry preparation step is an indispensable factor for efficiently producing a polyester. A terephthalic acid-alkylene glycol mixture with good slurry properties may be obtained by using an alkylene glycol in an excess large amount with respect to a terephthalic acid. However, when an alkylene glycol is present in an excess amount, the amount of side-reaction products generated by the polycondensation reaction is increased and this gives rise to a reduction in the melting point or polymerization degree of the resultant polyester and further causes coloration of the polyester. These problems may be avoided by using an alkylene glycol in an amount as close as possible to the chemically theoretical amount, but when the amount of an alkylene glycol used is decreased, a large amount of power is required for stirring the slurry in a preparation tank or a reaction vessel, or the slurry fluidity or reactivity in the esterification step is decreased, and there arises a problem that the esterification or polycondensation reaction takes a long time to complete. Accordingly, a slurry in which the amount of an alkylene glycol used is a required minimum amount with respect to the amount of a terephthalic acid used and which has good fluidity and high polycondensation reactivity, is suitable as a raw material for the production of a polyester by a direct polymerization method. For this purpose, it is important to use an alkylene glycol in an amount as small as possible and form a slurry with low viscosity and excellent stability in the slurry-forming step.

In order to attain the purpose of producing polyethylene terephthalate and other polyesters, a production process of a mixture of terephthalic acid with an alkylene glycol having good slurry properties is discussed in many patent publications, but in these publications, the technical interest is substantially focused on controlling the shape or particle size of the terephthalic acid particle.

Various methods for industrially producing a terephthalic acid are known, and one of these is a method of hydrolyzing dimethyl terephthalate as a starting material with water (see, for example, Patent Documents 1, 2 and 3).

As regards the method for controlling the particle diameter of terephthalic acid, various proposals have been made. A known representative example thereof is a method of liquid-phase oxidizing paraxylene as a starting material in a lower fatty acid solvent in the presence of a catalyst containing a transition metal compound and bromine, and then stepwise cooling the solution in a serially-connected multistage recrystallization tank, where the particle diameter of terephthalic acid produced is controlled by controlling the conditions for precipitating the terephthalic acid dissolved in the solution. However, this method has a problem such that the conditions at the precipitation of a terephthalic acid crystal are established mainly for the purpose of controlling the impurity concentration in the terephthalic acid crystal and therefore, the operating range allowed for controlling the particle size distribution is narrow.

In the case of producing a terephthalic acid by hydrolyzing dimethyl terephthalate, the hydrolysis reaction is preformed under high pressure, but almost no impurity is generated during hydrolysis and when the raw material dimethyl terephthalate is purified to a high purity, a recrystallization operation using a plurality of crystallizations can be fundamentally dispensed with. If the terephthalic acid produced by the reaction is again dissolved under high-temperature high-pressure conditions and recrystallized, a lot of energy is consumed and this is economically disadvantageous.

Other than the above-described method in which the production conditions of a terephthalic acid crystal is controlled, a method of varying the particle size distribution by grinding the produced terephthalic acid crystals in a slurry state is known (see, for example, Patent Document 4). Furthermore, there have been proposed, for example, a method where a part of the terephthalic acid slurry is withdrawn, pulverized by a wet grinder and then returned into the remaining slurry, thereby controlling the particle diameter distribution and changing the slurry properties (see, for example, Patent Document 5); and a method where terephthalic acid particles having an average particle diameter of 100 µm or more and terephthalic acid particle having an average particle diameter of 50 µm or more are mixed with each other at a mass ratio of 70-85% : 30-15% (see, for example, Patent Document 6). The method containing a grinding treatment has a problem that the effect on reducing the diameter of the particles of terephthalic acid by a pump or the like is low and a long time is required to obtain the desired apparent density. Also, the method in which two types of terephthalic acid particles different in the particle diameter from each other are mixed with each other requires equipments for separately storing the two different types of terephthalic acid particles under different storing conditions from each other, and then mixing the two different types of terephthalic acid particles together, or an equipment for separately crystallizing the two different terephthalic acid particles, but these requirements make this method disadvantageous with regard to equipment cost.

It is known that the terephthalic acid produced by hydrolyzing a raw material comprising dimethyl terephthalate generally have an aggregation structure change in the particle diameter according to the reaction conditions under which the terephthalic acid is formed (see, for example, Patent Document 7). By making use of these properties, the inventors of the present invention have found that in the method in which dimethyl terephthalate is hydrolysed to form a terephthalic acid, the resultant reaction mixture is flash-cooled, and then the resultant terephthalic acid-containing slurry is subjected to a solid-liquid separation to produce a terephthalic acid, there is a fixed relationship between the amount of fine particles generated at the flash cooling and the viscosity of the resultant slurry. The inventors have also proposed a method of contiguously measuring the viscosity of the terephthalic acid-containing slurry immediately after the flash cooling and controlling the reaction mixture temperature immediately before flash cooling based on the degree of fluctuation in the viscosity (see, for example, Patent Document 8). However, this method has a problem in that the operating conditions must be controlled within a significantly narrow range, for example, the slurry concentration needs to be strictly controlled or the pressure must be precisely checked for controlling the temperature, and the diameter of the obtained terephthalic acid particles is readily varied due to fluctuation in the slurry concentration, temperature or pressure.
[Patent Document 1] JP-55-141433A
[Patent Document 2] JP-08-231464A
[Patent Document 3] German Patent No. 2,916,197
[Patent Document 4] JP-09-255619A
[Patent Document 5] JP-11-100350A
[Patent Document 6] JP-48-029735A
[Patent Document 7] JP-2003-128598A (Scope of Claim for Patent)
[Patent Document 8] JP-2005-023054A (Scope of Claim for Patent)

### DISCLOSURE OF THE INVENTION

### Problems to be Solved by the Invention

The present invention has been made by taking into consideration problems in conventional arts. In other words, an object of the present invention is to provide a process for producing a liquid mixture of terephthalic acid with an alkylene glycol by mixing at least one alkylene glycol with terephthalic acid produced by the hydrolysis of a dialkyl terephthalate (DAT) used as a raw material, where a liquid mixture of terephthalic acid and alkylene glycol having with good slurry properties, i.e., low slurry viscosity and good slurry stability, is prepared without using a complicated process.

Accordingly, the present invention provides a process for producing a liquid mixture of terephthalic acid with an alkylene glycol having slurry properties suitable for polymerization reaction of a terephthalic acid produced by the hydrolysis of DAT with the alkylene glycol, where the liquid mixture can be produced using easy operation control and simpler equipment.

### Means to Solve the Problems

As a result of intensive studies to attain the above-described object, the inventors of the present invention have accomplished the process of the present invention.

The process of the present invention for producing a liquid mixture of terephthalic acid with an alkylene glycol, comprises preparing a liquid mixture containing terephthalic acid produced by the hydrolysis of a dialkyl terephthalate and at least one type of alkylene glycol mixed with terephthalic acid and having a temperature of 100 to 130°C, and stirring the liquid mixture for from 15 minutes to 3 hours while maintaining the temperature of the liquid mixture within the above-mentioned temperature range, to control the properties of the resultant slurry.

In the process of the present invention, the mixing molar ratio of the alkylene glycol to terephthalic acid is preferably from 1.2 to 2.0.

In the process of the present invention, preferably, the alkylene glycol is heated at a temperature of 100 to 130°C and terephthalic acid is then mixed into the heated alkylene glycol.

In the process of the present invention, the time for which the liquid mixture of terephthalic acid with the alkylene glycol is maintained at a temperature of 100 to 130°C is preferably in the range of 30 minutes to 2 hours.

In the process of the present invention, terephthalic acid is preferably one collected, after the hydrolysis of the dialkyl terephthalate, by cooling the resultant reaction mixture to a temperature of 100°C or less, and then subjecting the cooled mixture to a solid-liquid separation step.

In the process of the present invention, the alkylene glycol is preferably selected from ethylene glycol, 1,3-propylene glycol and 1,4-butylene glycol.

According to the process of the present invention, terephthalic acid particles produced by hydrolyzing a dialkyl terephthalate as a starting material can be mixed with an alkylene glycol without applying a precise control of the particles by a recrystallization device using a plurality of crystallization tanks or by a grinding device, whereby the resultant terephthalic acid-alkylene glycol liquid mixture has good slurry properties (low slurry viscosity and good slurry stability) required as the raw material for polyester and equipment costs and energy for the process can be reduced.

### Best Mode for Carrying Out the Invention

The best mode for carrying out the present invention is described in detail below, and includes a preferred embodiment of the present invention, but the present invention is not limited to this embodiment.

### Preparation of Terephthalic Acid

As regards the terephthalic acid for use in the present invention, a terephthalic acid obtained by hydrolyzing a dialkyl terephthalate can be used in the process of the present invention without limitation in the hydrolysis conditions, etc. The dialkyl terephthalic acid includes dimethyl terephthalate, diethyl terephthalate, dipropyl terephthalate, dibutyl terephthalate and dihexyl terephthalate.

Above all, a terephthalic acid obtained from dimethyl terephthalate through hydrolysis thereof under high-temperature high-pressure conditions by establishing the reaction temperature to from 180 to 300°C, preferably from 230 to 260°C, and a reaction pressure to from 2.8 to 4.6 MPa (gauge pressure) is preferred. As is known, the by-product mainly comprising methanol, which is generated due to the hydrolysis reaction, is discharged in the state of a vapor from the reaction vessel and thus the reaction equilibrium is shifted to the product-increase side, whereby a high reaction rate can be realized. At this time, the amount of impurities in the resultant terephthalic acid from the reaction is preferably 1,000 ppm or less and is more preferably controlled such that the total amount of dimethyl terephthalate and monomethyl terephthalate contained as impurities becomes 1,000 ppm or less. After the completion of reaction, the reaction liquid at a high temperature under a high pressure is in the state of a slurry comprising a terephthalic acid particles precipitated during the reaction and water in which terephthalic acid is dissolved. In order to perform solid-liquid separation of this slurry, the slurry is cooled by a procedure such as a flash cooling procedure under the ambient atmospheric pressure until the liquid temperature decreases preferably to 100°C or less, more preferably from 80 to 100°C. After cooling, the slurry comprising a terephthalic acid and water is preferably subjected to solid-liquid separation in a centrifugal separator, etc. As for the solid-liquid separation step, there are specifically a normal centrifugal separation step or a filtration step using a filter. The obtained terephthalic acid is in a hydrous state and thus is generally dehydrated by a drying operation, but in the present invention, it can be arbitrarily selected to perform or not perform the drying. If the amount of water contained in the terephthalic acid becomes large, the viscosity of the slurry may decrease, but the precipitation rate of the solid content in the slurry is increased and the probability of causing a trouble in the slurry transportation tends to increase. The amount of water contained in the terephthalic acid is preferably 30 mass% or less based on the solid mass of the terephthalic acid after bone-drying.

In the terephthalic acid obtained by such a method, the amount of 4-carboxybenzaldehyde and paratoluic acid, which are impurities, is very small as compared with a terephthalic acid obtained starting from paraxylene through an oxidation reaction, and the catalyst (e.g., Co, Mn, Br element) used in the process of producing the terephthalic acid is not contained. Accordingly, use of a terephthalic acid obtained by the above-described hydrolysis method is to use a terephthalic acid having a small impurity content and this is important for the product quality control of a polyester product.

### Alkylene Glycol

The alkylene glycol which can be used in the present invention comprises at least one member selected from ethylene glycol, 1,3-propylene glycol, 1,4-butylene glycol and a mixture thereof. The alkylene glycol generated and recovered in the process of producing a polyester, that is, in the esterification and polycondensation steps in the case of mainly using a terephthalic acid for the raw material, and in the transesterification reaction and polycondensation steps in the case of using a lower dialkyl ester of dicarboxylic acid for the raw material, can be also used in the production process of the present invention. From the quality aspect of the polymer obtained by a polymerization reaction, the mixing ratio of alkylene glycol to terephthalic acid is, in terms of the molar ratio, preferably closer to 1.0 which is a theoretical amount. However, on the other hand, good fluidity in the reaction vessel at the polymerization reaction needs to be ensured and this good fluidity must be maintained. Considering the balance of these properties and in view of quality maintenance and energy consumption, the molar amount of the alkylene glycol used is preferably from 1.2 to 2.0 times the molar amount of terephthalic acid. If the molar ratio is less than this range, as described above, the fluidity of the liquid mixture of terephthalic acid and alkylene glycol is sometimes decreased, whereas if it exceeds the range above, a large amount of alkylene glycol must be removed from the reaction vessel at the polymerization reaction and an efficient operation of the polyester production procedure can be hardly attained.

In the process of reaching the present invention, the inventors of the present invention made particular studies on the cause of increase in the viscosity of the liquid mixture of terephthalic acid and alkylene glycol. As a result, it was found that the terephthalic acid obtained by hydrolyzing DAT had many fine pores in the surface thereof and exhibited a high specific surface area and a low bulk density. Also, when the aggregation state of the terephthalic acid or the diameter of the terephthalic acid particles is varied, the specific surface area of the terephthalic acid particles is greatly changed. Particularly, when the amount of the fine particles of the terephthalic acid increases, the specific surface area of the terephthalic acid particles increases. When a slurry is prepared from the above-mentioned terephthalic acid particles and an alkylene glycol, it was found that the amount of the alkylene glycol necessary to wet the surfaces of the terephthalic acid particles increases. For this reasons, when a slurry is prepared by mixing the above-mentioned terephthalic acid particles into a fixed amount of alkylene glycol, the amount of the alkylene glycol serving as a key member for controlling the fluidity property of the resultant slurry decreases in comparison with the case where terephthalic acid particles having a low specific surface area are employed, and as a result, the viscosity of the resultant slurry greatly increases.

The present inventors have made intensive studies by taking these findings into consideration and discovered that the slurry viscosity is greatly governed by the dispersion state of terephthalic acid particles in the slurry because the specific surface area of the terephthalic acid is greatly changed, although the terephthalic acid and alkylene glycol are not changed in the composition and the particle size distribution measured by a wet process is not changed between before and after the mixing treatment. Namely, it was found the inventors of the present invention that in the production of terephthalic acid by the hydrolysis of DAT, when the amount of fine particles of terephthalic acid increases, the fine terephthalic acid particles serves as a binder, and thus, after solid-liquid separation step, the resultant terephthalic acid particles are firmly aggregated, and thus, the aggregated state of the terephthalate acid particles does not change even by the drying operation, and does substantially not change by mixing the aggregated terephthalic acid particles into an alkylene glycol at low temperature; the aggregated state of the terephthalic acid particles can be changed only by increasing the mixing temperature into the alkylene glycol; and after changing the aggregated state of the terephthalic acid particles as mentioned above, even when the temperature of the resultant liquid mixture (slurry) is decreased to room temperature, the slurry viscosity of the liquid mixture can be maintained low.

In other words, the inventors of the present invention have found that the viscosity of the liquid mixture of terephthalic acid and an alkylene glycol can be controlled by controlling the aggregation dispersion state of the terephthalic acid particles in the liquid mixture of terephthalic acid and an alkylene glycol produced by mixing an alkylene glycol with a terephthalic acid particles obtained by the hydrolysis of dimethyl terephthalate as a raw material. Controlling the aggregation·dispersion state is to continue by stirring of the liquid mixture of terephthalic acid and the alkylene glycol under heating within a specific temperature range over a fixed time when preparing this mixture by mixing terephthalic acid particles into the alkylene glycol. Preferably, the alkylene glycol is used in an amount of 1.2 to 2.0 times by mole based on the molar amount of terephthalic acid. In the process of the present invention, a liquid mixture containing a terephthalic acid produced by the hydrolysis of a dialkyl terephthalate and at least one type of alkylene glycol mixed therewith and having a temperature of 100 to 130°C is prepared, and the slurry properties are then adjusted by stirring this mixed solution for 15 minutes to 3 hours while keeping the temperature within the above-described temperature range.

The alkylene glycol is preferably heated in advance before being mixed with the terephthalic acid, and the heating temperature is preferably from 100 to 130°C. Even when the alkylene glycol is not previously heated, the same effect as that in the present invention may be obtained by heating the slurry after the mixing it with the terephthalic acid, and keeping maintaining the predetermined heating temperature for a long time. However, this is disadvantageous in that the slurry after mixing needs to be heated and its handling is difficult; and in the case of using continuous production equipment, the equipment size in the slurry preparation step becomes large so as to satisfactorily ensure the time for which the heated state is maintained; and furthermore, heating of a highly viscous slurry involves a high risk of causing an equipment trouble and a very large heating device is required. If the heating temperature is too low, the effect of the present invention is not obtained, whereas if the heating temperature is excessively high, the reaction between the terephthalic acid and the alkylene glycol is initiated and the quality of the polyester polymer as a product is difficult to stably maintain. In the case where the terephthalic acid is hydrous, the alkylene glycol evaporates at a temperature of 130°C or more in association with evaporation of water and the molar ratio of terephthalic acid to alkylene glycol changes, as a result, the conditions of the polymerization reaction are not stabilized and there may arise an adverse effect on the polyester quality with high probability.

The method of mixing terephthalic acid with an alkylene glycol is not particularly limited, and for example, a method where these are directly charged together into a stirring tank and then mixed with stirring, and a method where these are previously mixed by a line mixer, etc., and then charged into a stirring tank, may be used. The stirring blade in the stirring tank is also not limited. The stirring tank must be set to the same temperature as the temperature at which the alkylene glycol is previously heated, and the heating with stirring needs to be maintained for 15 minutes to 3.0 hours. It is a commonsense that the alkylene glycol is decreased in the viscosity by heating it, but in the case of using a terephthalic acid obtained by the hydrolysis of DAT, if the slurry is in a slurry state of a high-concentration terephthalic acid-alkylene glycol mixture having a low proportion of alkylene glycol, a good terephthalic acid-alkylene glycol mixture cannot be obtained only by decreasing the viscosity of the liquid component, i.e., alkylene glycol, and a terephthalic acid-alkylene glycol mixture suitably usable in the polyester production process cannot be obtained.

In the process of the present invention, the slurry dispersion state is controlled by applying heat energy necessary for changing the dispersion or aggregation state. The change in the slurry dispersion state or slurry aggregation state can be adjusted by the amount of heat' energy applied, and a slurry having a desired viscosity can be prepared by the temperature setting and the time for which the stirring state is maintained. However, extreme reduction in the temperature or time for which the stirring state is maintained may incur shortage of the amount of heat energy applied. Also, if the temperature is excessively high, as described above, the reaction between the terephthalic acid and the alkylene glycol is initiated and this may give rise to worsening of the polyester polymer quality. Furthermore, if the time for which the stirring state under heating is maintained exceeds 3 hours, the device efficiency decreases, whereas if the time for which the stirring state under heating is maintained is less than 15 minutes, the slurry viscosity is disadvantageously not stabilized. Considering the device efficiency and the like, the temperature is preferably from 100 to 130°C, and the time for which the stirring state under heating is maintained is preferably from 30 minutes to 2.0 hours.

The liquid mixture of terephthalic acid with an alkylene glycol obtained in accordance with the process of the present invention can have a viscosity of 3.0 Pa·s or less even when cooled to room temperature, for example, 25°C, and the viscosity can be adjusted by changing the time for which the heat-stirring state is maintained, according to the process characteristics of the polyester production. Furthermore, the liquid mixture of terephthalic acid with an alkylene glycol produced in accordance with the present invention, can be directly used in the polymerization reaction for a polyester and when the process proceeds to the polymerization reaction without cooling the liquid mixture, not only the energy of the heating is unwasted but also the amount of the alkylene glycol used is small and therefore, the quality of the polyester obtained by the polymerization reaction is not affected.

### Examples

The present invention will be concretely illustrated below by referring to Examples, but the present invention is not limited to these Examples. Incidentally, the values in Examples were determined by the following methods.

### (a) Particle Size Distribution:

The particle diameter distribution of the terephthalic acid particles in the terephthalic acid-alkylene glycol liquid mixture was measured by a laser scattering particle size distribution analyzer, Microtrac VSR MT3000, manufactured by Nikkiso Co., Ltd. after removing the alkylene glycol in the liquid mixture by a drying operation and dispersing the remaining terephthalic acid particles in water to prepare a slurry. Also, the average particle diameter was determined from the particle diameter distribution. The particle diameter distribution and average particle diameter of the terephthalic acid particles before preparing the terephthalic acid-alkylene glycol liquid mixture were measured similarly after forming the obtained solid sample into a slurry by using water.

### (b) Slurry Viscosity:

A sample of the liquid mixture was stirred for 10 minutes to prepare a slurry having a predetermined slurry concentration, and then the stirring was then stopped. Immediately after stopping the stirring, the viscosity of the liquid mixture was measured using a B-type viscometer (Brookfield model-DV-III programmable rheometer), and the measured value was taken as the slurry viscosity.

### (c) Specific Surface Area by BET Method:

After drying the terephthalic acid particles, the specific surface area thereof was measured by MONOSORB manufactured by Yuasa-Ionics Co., Ltd.

### (d) Slurry Stability:

The ratio of the slurry viscosity after 5 seconds from the stopping of stirring to the slurry viscosity immediately after the stopping of stirring was evaluated as the slurry stability. That is, the slurry stability is represented by the following formula. When this value is 80% or more, the slurry stability was rated as "good".

Slurry stability = slurry viscosity after 5 seconds from the stopping of stirring/slurry viscosity immediately after the stopping of stirring

### [Example 1]

In a batch reaction vessel equipped with a stirrer, dimethyl terephthalate and pure water were charged and heated. When the liquid temperature reached 250°C, the valve of the distillation tube provided in the top cover of the reaction vessel was opened, whereby methanol produced as a by-product by the hydrolysis reaction of dimethyl terephthalate was removed by distillation together with water vapor, and at the same time, water equal to the amount of water removed by distillation was continuously fed into the reaction vessel. After the passing of a predetermined time, the liquid temperature in the reaction vessel was decreased to 230°C while continuing the operation of removing methanol by distillation, and the liquid in the reaction vessel was maintained at this temperature for 30 minutes. Thereafter, the internal liquid was taken out and flashed into a vessel under the ambient atmospheric pressure through a pipe, thereby cooling the reaction liquid. The operation conditions during the hydrolysis reaction were controlled such that in the reaction liquid before flashing, the ratio between the mass of reaction water and the mass of terephthalic acid became 1.0. The slurry comprising terephthalic acid and water after the flash cooling was separated by filtration, as a result, the water percentage in the resultant terephthalic acid was 20 mass%. Also, the specific surface area by BET of the terephthalic acid was 2.56 cm²/g.

The average particle diameter of the terephthalic acid particles was 130 µm, and the content of fine particles having a particle diameter of 45 µm or less was 20 mass%. The terephthalic acid particles were dried, and ethylene glycol and the dried terephthalic acid particles were weighed to give a molar ratio of 1.6 of ethylene glycol to terephthalic acid. After heating the ethylene glycol to 120°C, the terephthalic acid was mixed into the heated ethylene glycol, and the resultant liquid mixture was stirred while maintaining the temperature at 120°C for 30 minutes. Thereafter, the viscosity of the resultant liquid mixture was measured while maintaining the liquid mixture at the heating temperature. The measured viscosity was 0.8 Pa·s. Also, the liquid mixture after this measurement of viscosity was cooled to 25°C while stirring, and the viscosity was again measured and found to be 1.8 Pa·s, revealing that the stability of this slurry was 90%. The resultant slurry after the viscosity measurement was subjected to solid-liquid separation, and ethylene glycol as a liquid portion was then removed by a drying operation. In the remaining terephthalic acid particles, the average particle diameter was 131 µm, and the specific surface area by BET of the terephthalic acid particles was 1.23 cm²/g.

Furthermore, the terephthalic acid-ethylene glycol liquid mixture after the treatment as mentioned above was directly used for polymerization reaction, and the resultant polyester had good quality. The results are shown in Table 1.

### [Comparative Example 1]

The terephthalic acid particles obtained in Example 1 and ethylene glycol were mixed at a molar ratio of 1.6 similarly to Example 1 and after stirring the resultant liquid mixture for 30 minutes without heating, the viscosity was measured and found to be 9.5 Pa·s.

### [Examples 2 to 5 and Comparative Examples 2 and 3]

In each of Examples 2 to 5 and Comparative Examples 2 and 3, using 4 continuous tank-type reactors connected in series, 1 part by mass of dimethyl terephthalate and 1 part by mass of water were continuously fed into a first reaction tank, and hydrolysis was performed at a temperature of 220 to 255°C in each tank under a pressure of 2.3 to 4.5 MPa. The obtained reaction mixture was directly flash-cooled to recover the atmospheric pressure, and the resulting terephthalic acid-containing slurry was subjected to solid-liquid separation by a centrifugal separator to obtain a terephthalic acid particles. The water percentage in the terephthalic acid particles was 18 mass%. In the obtained terephthalic acid particles, the average particle diameter was 131 µm and the content of fine particles having a particle diameter of 45 µm or less was 14 mass%. A terephthalic acid-ethylene glycol liquid mixture was prepared and its viscosity was measured by the same procedures as in Example 1 except for directly using the above-obtained terephthalic acid particles without drying and changing the temperature at the heating of ethylene glycol, the temperature after mixing and the mixing time as shown in Table 1. Comparative Examples 2 and 3 were performed as in Examples 4 and 5, respectively, without applying heating, and the viscosity and slurry stability after stirring for the same time as in Examples 4 and 5 were measured at 25°C. The measurement results are shown in Table 1.

### [Example 6]

The same procedures as in Example 1 was performed except that in the hydrolysis reaction, the mass ratio of reaction water to terephthalic acid was 2.0 and 1,3-propylene glycol was used as an alkylene glycol, and the viscosity and slurry stability of the resultant terephthalic acid-propylene glycol liquid mixture were measured and found to be 2.0 Pa·s and 85%, respectively. The measurement results are shown in Table 1.

### [Example 7]

A terephthalic acid was prepared in the same manner as in Example 1 and naturally cooled to 100°C under the ambient atmospheric pressure without applying flash cooling. Thereafter, the terephthalic acid slurry was taken out and subjected to solid-liquid separation. In the obtained terephthalic acid particles, the average particle diameter was 200 µm and the percentage of giant particles having a particle diameter of 250 µm or more was 30 mass%. This terephthalic acid particles were dried, and ethylene glycol and the dried terephthalic acid particles were weighed at 25°C to give a molar ratio of 1.6 of ethylene glycol to terephthalic acid, and the terephthalic acid particles were mixed, into ethylene glycol and as a result, a clay-like mixture was formed and the viscosity of the mixture as a slurry could be hardly measured. On the other hand, ethylene glycol was heated to 120°C and mixed with the terephthalic acid particles at the same molar ratio as mentioned, and the mixture was stirred for 30 minutes while maintaining the temperature at the above-mentioned level and then cooled. The viscosity and stability of the resultant slurry were measured and found to be 3.0 Pa·s and 85%, respectively. The measurement results are shown in Table 1.

**Table 1**

| | Example 1 | Comparative Example 1 | Example 2 | Example 3 | Example 4 | Example 5 | Compar ative Example 2 | Compar ative Example 3 | Example 6 | Example 7 |
|---|---|---|---|---|---|---|---|---|---|---|
| Glycol/TPA^{*1} | 1.6 | 1.6 | 1.6 | 1.6 | 1.2 | 1.8 | 1.2 | 1.8 | 1.6 | 1.6 |
| Type of glycol | EG | EG | EG | EG | EG | EG | EG | EG | PG | EG |
| Temperature of glycol (°C) | 120 | 25 | 100 | 130 | 120 | 120 | 25 | 25 | 120 | 120 |
| Temperature after mixing with stirring (°C) | 120 | 25 | 100 | 130 | 120 | 120 | 25 | 25 | 120 | 120 |
| Time for which heat-stirring is continued (min) | 30 | 30 | 90 | 40 | 60 | 60 | 60 | 60 | 30 | 30 |
| Temperature (°C) at slurry viscosity measurement | 25 | 25 | 25 | 130 | 25 | 25 | 25 | 25 | 25 | 25 |
| Slurry viscosity (Pa·s) | 1.8 | 9.5 | 2.0 | 1.0 | 2.5 | 1.5 | 10.1 | 8.3 | 2.0 | 3.0 |
| Slurry stability (%) | 90 | 95 | 89 | 86 | 89 | 88 | 95 | 83 | 85 | 85 |

| | | | | | | | | | | |
|---|---|---|---|---|---|---|---|---|---|---|
| [Note] *1: Molar ratio of glycol to terephthalic acid TPA: terephthalic acid, EG: ethylene glycol, and PG: 1,3-propylene glycol. | | | | | | | | | | |

### INDUSTRIAL APPLICABILITY

In the process of the present invention, a terephthalic acid particle produced by a hydrolysis method starting from dimethyl terephthalate is mixed with an alkylene glycol without applying a precise control of the particles by a recrystallization device using a plurality of crystallization tanks or by a grinding device, so that a terephthalic acid-alkylene glycol liquid mixture having good slurry properties and suitable as a raw material for polyester can be prepared and the costs of equipment and energy can be reduced. Accordingly, the present invention has great industrial significance in the manufacture of a polyester.

## Claims

1. A process for producing a liquid mixture of terephthalic acid with an alkylene glycol, comprising preparing a liquid mixture containing terephthalic acid produced by the hydrolysis of a dialkyl terephthalate and at least one type of alkylene glycol mixed with terephthalic acid and having a temperature of 100 to 130°C, and stirring the liquid mixture for from 15 minutes to 3 hours while maintaining the temperature of the liquid mixture within the above-mentioned temperature range, to control the slurry properties of the resultant liquid mixture.

2. The process as claimed in claim 1, wherein the mixing molar ratio of the alkylene glycol to terephthalic acid is from 1.2 to 2.0.

3. The process as claimed in claim 1 or 2, wherein the alkylene glycol is heated at a temperature of 100 to 130°C and terephthalic acid is then mixed into the heated alkylene glycol.

4. The process as claimed in claim 1 or 2, wherein the time for which the liquid mixture of terephthalic acid with the alkylene glycol is maintained at a temperature of 100 to 130°C is in the range of 30 minutes to 2 hours.

5. The process as claimed in claim 1 or 2, wherein terephthalic acid is one collected, after the hydrolysis of the dialkyl terephthalate, by cooling the resultant reaction mixture to a temperature of 100°C or less, and then subjecting the cooled mixture to a solid-liquid separation step.

6. The process as claimed in claim 1 or 2, wherein the alkylene glycol is selected from ethylene glycol, 1,3-propylene glycol and 1,4-butylene glycol.
